# EUROPEAN PATENT APPLICATION

(11) **EP 1 279 389 A2**
(43) Date of publication of application: **29.01.2003**
(21) Application number: 02425481.5
(22) Date of filing: 24.07.2002
(51) Int. Cl.: A61F 13/15

(54) **A feed unit supplying strip material to a line for the manufacture of personal hygiene items**

(30) Priority: 24.07.2001 IT BO20010470
(71) Applicant: GDM S.p.A., 40133 Bologna (IT)
(72) Inventor: Kastriot, Shqypi, 26010 Bagnolo,(Cremona) (IT); Gandini, Mario, 20148 Milano (IT)
(74) Representative: Lanzoni, Luciano

(57) **Abstract**

A feed unit (14) supplying strip material to a line (L1) for the manufacture of personal hygiene items, typically nappies, sanitary towels and such like, is equipped with a first driving member (27) and a second driving member (28) coordinated one with another in such a manner as to engage a first strip (15) and a second strip (16) of absorbent material in alternation, according to a set cycle, and direct them respectively into a mill (12) by which they are pulped.

## Description

The present invention relates to a feed unit by which strip material is supplied to a line for the manufacture of personal hygiene items.

The invention finds application to advantage in the manufacture of absorbent paddings for personal hygiene products, namely nappies, sanitary towels and the like.

Accordingly, reference will be made explicitly in the following specification to units or lines for the manufacture of absorbent paddings utilized in nappies, albeit with no limitation implied.

Conventionally, disposable nappies comprise an absorbent padding sandwiched normally between a permeable inner layer of nonwoven fabric and an impermeable outer layer of polyethylene.

Formerly, absorbent paddings for nappies were relatively thick and would be obtained by cutting a continuous strip consisting in an aggregation of absorbent material, typically cellulose fibres, into discrete lengths.

The current practice is to make such absorbent padding from cellulose that has been defibrated, not least in order to facilitate the production of different shapes. In other words, sheet cellulose material is processed in such a way as to obtain a homogeneous distribution of fibres and air. This processing operation takes place normally in a pulp mill; continuous cellulose strip decoiling from a roll is directed into the inlet of the mill and emerges from the outlet broken down into relatively small particles, from which the absorbent padding is then fashioned.

To ensure that the lines on which these absorbent paddings are prepared can operate continuously, an uninterrupted supply of cellulose material must be provided, typically by replacing each roll with a new roll as it nears depletion.

Conventionally, the passage or changeover from the depleting strip to the new strip is brought about by overlapping the leading end of the new roll with the trailing end of the depleting roll and splicing the two together with adhesive film.

This operation betrays various drawbacks.

A first drawback, deriving from the fact that the overlapping portions of the two strips are of a certain length, is that with the mill momentarily receiving double the normal quantity of cellulose, there are clear risks of congestion at the pulping stage.

Another drawback derives from the presence of the elastic splicing film, which is typically a plastic and thus constitutes foreign matter once inside the mill, besides representing a fire hazard given the possibility of combustion in close proximity to a readily inflammable material such as cellulose.

The object of the present invention is to provide a feed unit for strip material that is unaffected by the aforementioned drawbacks and at the same time functional, economically advantageous and of simple embodiment.

The stated object is realized, according to the present invention, in a feed unit supplying strip material to a line for the manufacture of personal hygiene items, wherein at least a first strip and a second strip of material are directed respectively along a first feed path and a second feed path into a processing station of the line, characterized in that it comprises means by which the first strip and the second strip are taken up in alternation, separately one from the other and according to a predetermined cycle, and fed sequentially to the processing station.

The invention will now be described in detail, by way of example, with the aid of the accompanying drawings, in which:
- figure 1 is the illustration of a feed unit for strip material according to the present invention, viewed schematically in elevation and in a first operating configuration;
- figure 2 shows the unit of figure 1 in a different operating configuration;
- figure 3 illustrates an alternative embodiment of the unit in figure 1, viewed schematically and in elevation.

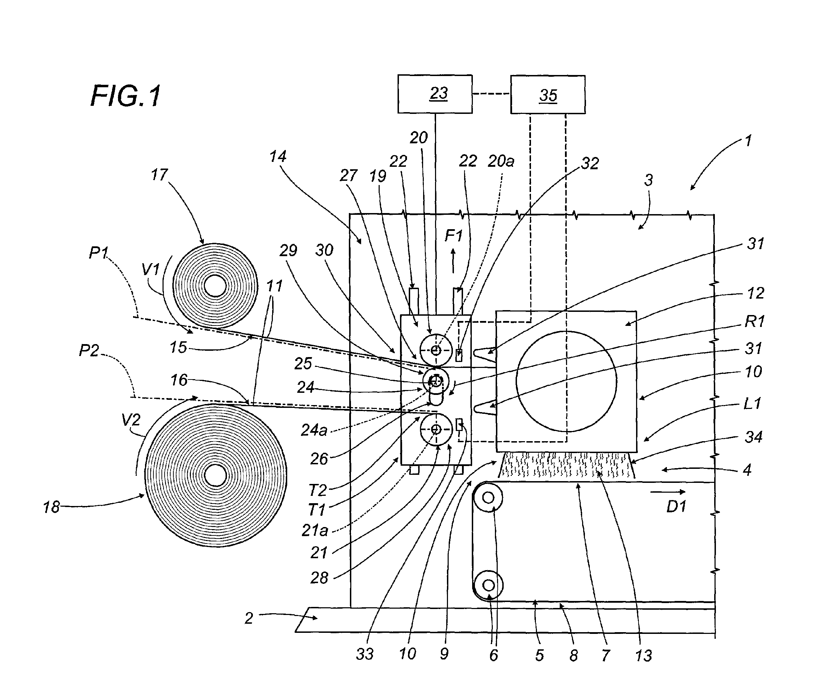

With reference to figure 1 of the accompanying drawings, 1 denotes a portion of a machine for the manufacture of absorbent paddings as utilized in personal hygiene items, particularly nappies (not illustrated)

The machine 1 comprises a frame 2, and a vertical bulkhead 3 supported by the frame.

The bottom part of the machine 1 incorporates a conveyor 4 consisting in an endless belt element 5 looped around a plurality of pulleys 6, carried by the bulkhead 3 and rotatable thus about horizontal axes, of which two only are indicated and of which at least one is driven by a motor (not illustrated) in such a way that the belt element 5 can be made to advance in a predetermined feed direction D1.

The belt element 5 affords a plurality of pockets (not illustrated) distributed uniformly and at a predetermined pitch along the entire developable length of the selfsame element 5, internally of which the aforementioned paddings are fashioned.

The pulleys 6 and the belt element 5 combine to create an active top branch 7, serving to establish a line L1 along which the paddings are formed and transported, and a return bottom branch 8.

The machine 1 comprises a station 10, coinciding with an infeed end 9 of the aforementioned line L1, of which the function is to process an absorbent strip material 11.

The station 10 incorporates a pulp mill 12 of conventional embodiment capable of breaking up the strip 11 up into fibrous particles 13 that will be formed subsequently into absorbent paddings (not illustrated) as they pass along the line L1.

In particular, the absorbent strip material 11 consists in a strip of cellulose.

The machine 1 also comprises a feed unit 14 by which the strip material 11 is directed into the processing station 10.

The feed unit 14 is in receipt of a first and a second strip of absorbent material, denoted 15 and 16 respectively, decoiled from respective rolls 17 and 18 in respective directions denoted V1 and V2.

The strips 15 and 16 decoiling from the rolls 17 and 18 pass along respective first and second paths denoted P1 and P2.

Interposed between the rolls 17 and 18 and the pulp mill 12, the feed unit 14 comprises a plate 19 carrying a first and a second power driven roller, both of conventional type, denoted 20 and 21.

The plate 19, of rectangular outline, is coupled slidably with two rectilinear ways 22 secured to the vertical bulkhead 3 and extending perpendicular to the frame 2.

The plate 19 is capable of rectilinear movement on the ways 22, generated through the agency of a conventional actuator indicated schematically in the drawings by a block denoted 23.

The rollers 20 and 21 rotate about respective horizontal axes 20a and 21a disposed substantially normal to the plate 19 and to the viewing plane of figure 1.

The unit 14 further comprises an idle roller 24 rotatable about an axis 24a disposed parallel to the axes 20a and 21a of the power driven rollers 20 and 21, which is located between the selfsame two rollers 20 and 21 and carried freely by a relative shaft 25 associated immovably with the vertical bulkhead 3.

The shaft 25 of the idle roller 24 is inserted through a slot 26 located between the two power driven rollers 20 and 21 and extending parallel to the aforementioned rectilinear ways 22, in such a manner that the rectilinear motion of the plate 19 along the ways 22 can be rendered independent of the shaft 25, which remains fixed relative to the vertical bulkhead 3.

The power driven rollers 20 and 21 function as respective first and second driving members 27 and 28 for the first and second strips 15 and 16.

The idle roller 24 functions as a guidance and pinch member 29 for the first and second strips 15 and 16.

The plate 19, the power driven rollers 20 and 21 and the idle roller 24 together constitute means 30 by which to take up and feed the strips 15 and 16 decoiling from the relative rolls 17 and 18.

Also forming part of the machine 1 are conveying elements 31, located between the power driven rollers 20 and 21 and the mill 12, by which the strips 15 and 16 are directed into the mill.

At a given point downstream of the power driven rollers 20 and 21, in the decoiling direction V1 and V2, the feed unit 14 comprises two sensors 32 and 33 located in close proximity respectively to the first power driven roller 20 and to the second power driven roller 21 and serving to detect the presence or absence of the strip 15 and 16 at a point immediately beyond the respective roller 20 or 21.

The fibrous particles 13 of absorbent material 11 are discharged by the mill 12 into the pockets on the active top branch 7 of the conveyor 4 by way of a duct 34 and with the aid of conventional suction devices (not illustrated) located on the opposite side of the belt element 5.

In operation, the belt element 5 advances along the feed direction D1 and, during its progress, the pockets in which the paddings are formed advance likewise along the relative line L1, passing in succession beneath the duct 34 of the mill 12 from which a layer of fibrous particles 13 generated by processing the absorbent material 11 is deposited into each pocket with the aid of the aforementioned suction means (not illustrated).

The suction means are able to exert an aspirating action on the fibrous particles 13 by virtue of the fact that the belt element 5 of the conveyor 4 is fashioned from a material permeable to air.

The steps in which the absorbent material 11 is reduced to fibrous particles 13 and dispensed by the mill 12 are familiar to a person skilled in the art and therefore require no further description.

Departing from the configuration of figure 1, the first strip 15 is pinched between the first power driven roller 20 and the idle roller 24 and fed thus to the inlet of the mill 12, decoiling from the relative roll 17 in the direction denoted V1.

The plate 19 occupies a first lowered position T1 in which the first power driven roller 20 and the idle roller 24 are mutually tangential.

In this first position T1, the first power driven roller 20 rotates in the same direction as that of the first roll 17, i.e. the decoiling direction V1, whilst the idle roller 24 rotates in the direction denoted R1 in figure 1, i.e. clockwise.

Accordingly, the elements of the take-up and feed means 30 assume a first configuration of engagement with the first strip 15, which is thus directed into the mill 12 of the processing station 10, and at the same time of substantial disengagement from the second strip 16, which simply rests against the second power driven roller 21 occupying a standby position T2.

Following the depletion of the first strip 15 decoiling from the first roll 17, the sensor 32 located next to the first power driven roller 20 will relay a signal to a monitoring and control unit 35 indicating the end-of-roll condition.

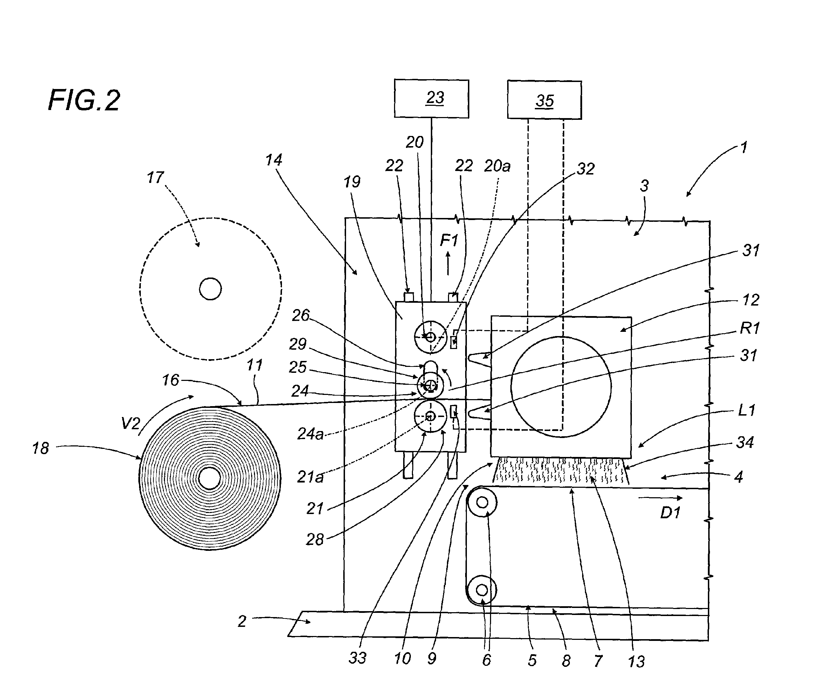

As illustrated in figure 2, the control unit 35 activates the actuator 23, which will respond by raising the plate 19 in the direction of the arrow denoted F1 so as to distance the first power driven roller 20 from the idle roller 24 and cause the second power driven roller 21, with the leading end of the new strip 16 already in place and occupying the standby position T2, to approach the selfsame idle roller 24.

The second power driven roller 21 is now set in rotation, causing the second strip 16 to decoil from the second roll 18 in the direction denoted V2 and advance toward the inlet of the mill 12.

As discernible in figure 2 and from the foregoing description, the take-up and feed means 30 are now made to assume a second configuration of engagement with the second strip 16, which is thus directed into the mill 12 of the processing station 10, and at the same time of substantial disengagement from the first strip 15, which is missing momentarily but, as soon as a new roll has been fitted to the machine 1, will be simply rested against the first power driven roller 20 in a position corresponding to the standby position T2 described previously with reference to figure 1.

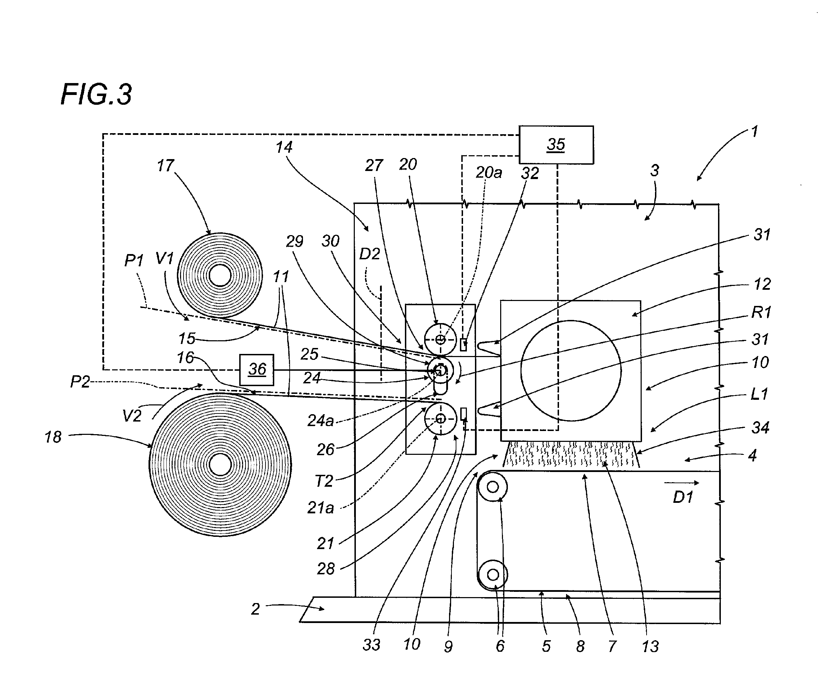

In the alternative embodiment of the present invention illustrated in figure 3, the plate 19 carrying the rollers 20 and 21 is fixed in relation to the vertical bulkhead 3 of the machine 1 whereas the idle roller 24 is rendered capable of movement, through the agency of a relative actuator 36 and in a direction perpendicular to the frame 2, between two different positions of engagement respectively with the first power driven roller 20 and with the second power driven roller 21, in which the first strip 15 and the second strip 16 respectively are fed into the mill 12. Figure 3 shows the position in which the idle roller 24 is in engagement with the first power driven roller 20.

With reference to both embodiments of the present invention described in the foregoing specification, the devices by which power is transmitted to the rollers 20 and 21 are conventional and therefore not illustrated.

To advantage, the feed unit will comprise suction means of conventional embodiment, not illustrated, by which the leading end of the new strip 15 or 16 is held in the standby position T2 on or near the relative power driven roller 20 or 21.

## Claims

1. A feed unit supplying strip material to a line for the manufacture of personal hygiene items, wherein at least a first strip (15) and a second strip (16) of material (11) are directed respectively along a first feed path (P1) and a second feed path (P2) into a processing station (10) of the line (L1), **characterized in that** it comprises means (30) by which the first strip (15) and the second strip (16) are taken up in alternation, separately one from the other and according to a predetermined cycle, and fed sequentially to the processing station (10).

2. A unit as in claim 1, wherein the strips (15, 16) and the take-up and feed means (30) are caused to assume a first configuration one relative to the other in which the take-up and feed means (30) engage the first strip (15) so as to direct the selfsame strip into the processing station (10), while remaining disengaged at least in part from the second strip (16), also a second configuration one relative to the other in which the take-up and feed means (30) engage the second strip (16) so as to direct the selfsame strip into the processing station (10), while remaining disengaged at least in part from the first strip (15).

3. A unit as in claim 2, operating in conjunction with respective devices (17, 18) for temporary storage of the first strip (15) and the second strip (16), wherein the passage of the strips (15, 16) and the take-up and feed means (30) from one configuration to the other occurs at a moment coinciding exactly or approximately with the full depletion of the relative strip (15, 16) supplied from one of the temporary storage devices (17, 18).

4. A unit as in claims 1 to 3, wherein at the same time as one of the two strips (15, 16) is directed into the processing station (10) by the take-up and feed means (30), the other strip (16, 15) remains substantially motionless in a standby position (T2) of readiness to be engaged by the take-up and feed means (60) according to the predetermined cycle.

5. A unit as in claims 1 to 4, wherein the take-up and feed means (30) include a first driving member (27) and a second driving member (28) engaging the first strip (15) and the second strip (16) respectively, and at least one guidance and pinch member (29) engaging the selfsame strips, of which the first and second driving members (27, 28) are designed to interact in alternation with the guidance and pinch member (29) when the take-up and feed means assume the first and the second configuration.

6. A machine as in claim 5, wherein the guidance and pinch member (29) is interposed between the first driving member (27) and the second driving member (28), and the first feed path (P1) and second feed path (P2) are interposed respectively between the first driving member (27) and the guidance and pinch member (29) and between the guidance and pinch member (29) and the second driving member (28).

7. A unit as in claim 5 or 6, wherein the first and second driving members (27, 28) comprise respective power driven rollers (20, 21).

8. A unit as in claims 5 to 7, wherein the guidance and pinch member (29) comprises an idle roller (24) .

9. A unit as in claims 5 to 8, wherein the first and second driving members (27, 28) are carried by a support element (19) capable of movement, generated by respective actuator means (23), relative to the guidance and pinch member (29).

10. A unit as in claims 5 to 8, wherein the guidance and pinch member (29) is capable of movement, generated by respective actuator means (36), relative to the first and second driving members (27, 28).

11. A unit as in claims 5 to 10, comprising means (32, 33) by which to sense the depletion of at least one of the strips (15, 16).

12. A unit as in claim 11, wherein the sensing means (32, 33) are interposed between the driving members (27, 18) and the processing station (10).

13. A unit as in claim 11 or 12, comprising means (35), connected to the sensing means (32, 33) and serving to monitor and control the operation of the take-up and feed means (30).
